**Europäisches Patentamt**

**European Patent Office**

**Office européen des brevets**

(11) Publication number : **0 360 812 B1**

# (12) EUROPEAN PATENT SPECIFICATION

(45) Date of publication of patent specification :
29.01.92 Bulletin 92/05

(51) Int. Cl.⁵ : **B29C 35/08, B29C 41/14**

(21) Application number : **88903895.6**

(22) Date of filing : **04.05.88**

(86) International application number :
**PCT/GB88/00347**

(87) International publication number :
**WO 88/09092 17.11.88 Gazette 88/25**

(54) **METHOD AND APPARATUS FOR PRODUCING AN ARTICLE BY MICROWAVE HEATING.**

(30) Priority : **07.05.87 GB 8710833**

(43) Date of publication of application :
**04.04.90 Bulletin 90/14**

(45) Publication of the grant of the patent :
**29.01.92 Bulletin 92/05**

(84) Designated Contracting States :
**AT BE CH DE FR GB IT LI LU NL SE**

(56) References cited :
**FR-A- 2 453 403**
**FR-A- 2 520 114**
**IEEE Instrumentation and Measurement Conference, 25-27 March 1986, Boulder, Colorado, IEEE (New York, US). P. Wendland : "Fiber optic measurements. A review of recent methodology", pages 219-222a.**

(56) References cited :
**Patent Abstracts of Japan, vol. 4, no 175 (E-36) (657), 3 December 1980, & JP,A, 55120240 (HITACHI SEISAKUSHO K.K.) 16 September 1980**

(73) Proprietor : **POROUS PLASTICS LIMITED**
**Pavilion 3 Little Park Farm Road, Segensworth Fareham Hampshire PO15 5TB (GB)**

(72) Inventor : **DAVIDSON, Roderick, Iain**
**Pavilion 3, Little Park Farm Road**
**Segensworth Fareham Hampshire PO15 5TB (GB)**
Inventor : **HORNSBY, Peter, Ridsdale**
**31 Manor Way Chesham**
**Buckinghamshire HP5 3BH (GB)**

(74) Representative : **Boff, James Charles**
**c/o Phillips & Leigh 7 Staple Inn Holborn**
**London WC1V 7QF (GB)**

## Description

This invention concerns a method and apparatus for producing an article, such, for example, as a condom or a rubber glove, by microwave heating.

In GB-A-1,310,697 there is disclosed a process for producing a pharmaceutical capsule shell in which a former having an electrically conductive outer surface is dipped into an aqueous thermal gelling coating solution so as to coat it therewith, induction heating of the former is effected so as to gel the coating, and the gelled coating is then dried in a warm air oven. Such a process therefore requires a two-stage heating procedure and does not lend itself to automation since a separate induction heating coil would appear to be required for each former used.

The present invention provides a method of producing an article comprising the steps of:-

i) covering an external surface of at least a portion of a former with liquid-containing material;

ii) providing heating so as to leave a dry solid coating on said former; and

iii) effective relative separation between the former and the dry solid coating so as to produce the article;

characterised in that the former is a microwave-heatable former which is heated by being subjected to microwave radiation so as to produce the said dry solid coating.

In the case of the present invention in contrast to the prior art referred to above, only one heating step is required, and this heating step can be readily controlled by varying the power input. When the liquid-containing material contains water (as in a rubber latex) or is a microwave receptive material (e.g. a PVC plastisol), the microwave energy simultaneously heats up both the former and the coating thereon of the liquid-containing material, so effecting drying and/or accelerating gelation.

In the case of the present invention, moreover, automation can easily be achieved since a large number of formers can be heated together within a common multimode microwave oven.

The liquid-containing material may be in the form of a solution, suspension or slurry.

The former is preferably made at least predominantly of ceramic material such, for example, as glass or silicon carbide.

The liquid-containing material, moreover, may itself be microwave-heatable.

As indicated above, the liquid-containing material may be a curable or gellable material, e.g. a latex solution or a plastisol.

The microwave radiation may be first employed to raise the liquid-containing material to a temperature at which the material becomes substantially solid, the microwave radiation being thereafter employed to raise the substantially solid material to a higher temperature at which it will be cured or gelled.

The article may be a hollow article. For example, the hollow article may be made of insulating material, the former being electrically conductive; the hollow article, while still on the former, being tested for pinholes therein by maintaining the solid coating on the former in contact with a body of electrically conductive liquid which is at a different potential from that of the former, and noting any factor related to a change in the potential difference between the potential of the former and that of the body of liquid.

The testing may be carried out by introducing the former, with the solid coating thereon, into an earthed tank containing water, and applying a voltage to the former.

The former may be porous, in which case, after the solid coating has been formed on the porous former, a gas may be passed through the latter so as to assist in removing the solid coating therefrom.

The invention also comprises apparatus comprising:-

i) a former;

ii) means for covering an external surface of at least a portion of the former with liquid-containing material;

iii) heating means for removing liquid from said material and so as to leave a dry solid coating on said former; and

iv) means for effecting relative separation between the former and the dry solid coating so as to produce the article;

characterised in that the former is a microwave-heatable former and the heating means are arranged to heat the former by subjecting it to microwave radiation so as to produce the said dry solid coating.

The invention is illustrated, merely by way of example, in the accompanying diagrammatic drawing which illustrates the production of a condom by the method and apparatus of the present invention.

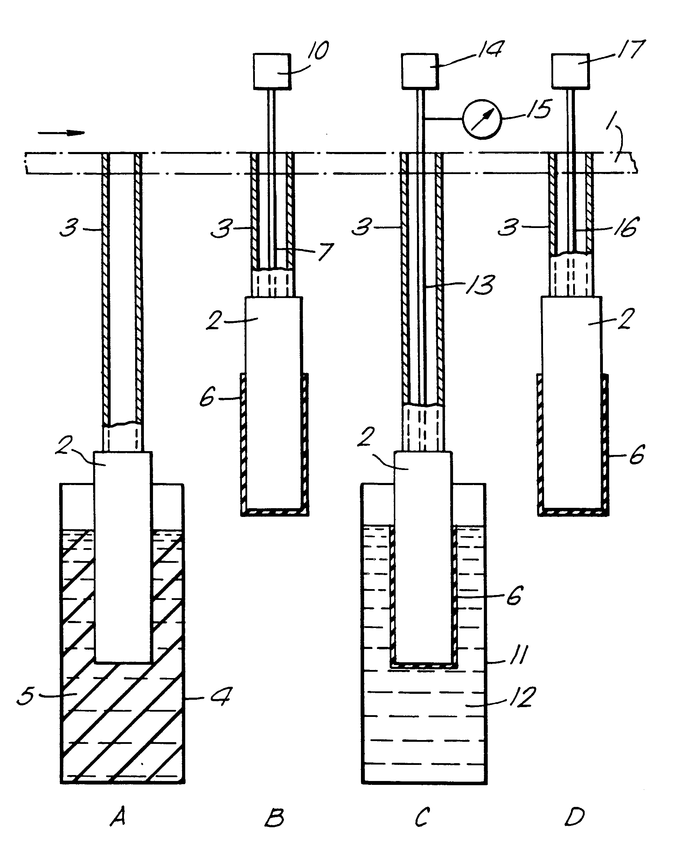

Referring to the drawing, an endless, horizontally disposed, support chain 1 supports a plurality of equally spaced apart formers 2 each of which is supported from the chain 1 by a hollow tubular member 3 which is arranged to be raised and lowered (by means not shown). The chain 1 is movable by a motor (not shown) so that each of the formers 2 is moved successively from a latex investment station A to a microwave heating station B to a pinhole testing station C and to a separation station D.

Each of the formers 2 is a porous, electrically conductive microwave-heatable member which is preferably made predominantly of a ceramic material such as silicon carbide or glass. Excellent results can be produced by the use of SILMOR silicon carbide. (SILMOR is a trade mark of Morganite Special Carbons Limited). SILMOR silicon carbide material is produced by the conversion of a mass of porous carbon or graphite to β -silicon carbide by reaction of the mass with silicon monoxide vapour whereby the surface carbon of said mass reacts with the silicon monoxide vapour to form silicon carbide in situ. This material, has high thermal conductivity and thermal shock resistance allowing rapid heating and cooling without hot-spots developing and has desirable surface finish and other characteristics. The material has the following specification:-

| | |
|---|---|
| Bulk Density | $2230 \ kg \ m^{-3}$ |
| Converstion depth | Up to 5mm |
| Hardness | 12+ (Moh's scale) |
| Temperature limit of operation | $1600^{\circ}C$ |
| Thermal expansion | $3.8\text{-}4.6 \times 10^{-6} \ ^{\circ}C^{-1}$ $(25\text{-}1000^{\circ}C)$ |
| Bend Strength | $89MN \ m^{-2}$ |
| Young's Modulus | $106 \ GN \ m^{-2}$ |
| Thermal conductivity | $100W \ m^{-1} \ K^{-1}$ |

An alternative is to use cast reconstituted silicon carbide to make the formers. Further alternatives are to use sintered β -silicon carbide, which has an excellent rate of heating; or microwave receptive glass or slip-castable ceramics uniformly doped with microwave receptive additives, such as silicon carbide or tin oxide.

At the late investment station A, each former 2 is lowered into a tank 4 containing rubber latex solution 5 so as to receive a latex investment 6. Alternatively, and depending upon the article being produced, the tank 4 may contain a PVC plastisol (i.e. a suspension of PVC particles in a plasticizer) with or without inclusion of microwave-receptive additives, such as carbon black, aluminium silicate, a metal oxide such as $Fe_3O_4$ or a polar liquid additive such as ethylene glycol. Such additives may be used to enhance the heating rate when the former 2 is subjected to microwave radiation at the microwave heating station B.

At the microwave heating station B, the former 2 is subjected, by way of a waveguide 7 introduced through the respective hollow tubular member 3, to microwave radiation generated by a microwave radiation generator 10. The temperature of the rubber latex of the latex investment 6 is first raised rapidly to about 95°C to remove the water and then, after a predetermined time period, is raised to 130°C to effect full cure of the rubber. If a plastisol is used in the tank 4, the microwave heating similarly effects gelling of the plastisol.In either case, the microwave energy employed is particularly effective in removing the water since the latter is receptive to microwave energy and therefore rapidly reaches its boiling point and evaporates.

At the pinhole testing station C, the former 2, with the now solid latex investment 6 thereon, is lowered into an earthed tank 11 containing water 12. The former 2 is connected by a conductor 13 to a stabilised voltage source 14, the voltage applied to the former 2 being indicated on a meter 15. If there is a hole through the latex investment 6, the voltage will be lower than would otherwise be the case, and the reading provided by the meter 15 will therefore provide an indication as to whether such a pinhole exists. Although for illustrative purposes a meter 15 is shown in the drawing, electronic equipment (not shown) may be employed to provide an alarm and to stop the process.

At the separation station D, the porous former 2 is connected by a pipe 16, which is introduced through the tubular member 3, to a source 17 of compressed air so that the latter can pass to the interface between the former 2 and the now fully formed condom 6 to render easier the separation of the latter (by means not shown) from the former 2. As will be appreciated, if compressed air is used in this way, it is important to ensure that the latex does not become keyed in the pores of the former so as to block the pores. This may be effected by control of pore size and/or the use of a non-toxic release agent on the surface of the former prior to its investment with the latex.

The use of microwave-heatable formers 2 in the method described above permits a reduction in the cycle time since the formers 2 can be heated and cooled much more rapidly than those heated by other methods. Since, moreover, the formers 2 are electrically conductive, it is not necessary to rerove the finished condoms from them and mount them on separate supports in order to test them.

The use of a porous former, in addition to rendering it possible to use the method described in the preceding paragraph, also allows the possibility of increased cooling rates after the former has passed through the microwave heating station B. Additionally, the use of a porous former to produce a hollow product enables the latter to be tested for pinholes by inflating it under internal air pressure whilst it is still attached to the former. Such a procedure could be used in substitution for the testing method described above. However, it will of course be appreciated that the formers 2 are not necessarily porous.

The method illustrated in the drawing is also suitable for the production of other hollow articles, such as rubber gloves. However, the articles which can be produced by the method of the present invention are not necessarily hollow since sheet articles such as thick section plasticised PVC components may also be produced thereby. Furthermore, the method can be used to produce foamed plasticised PVC or expanded rubber parts, using air entrainment methods (frothing) or chemical blowing agents to yield a porous internal structure.

When the liquid-containing material used in the present invention is a rubber latex solution, the heating effected by the former causes water vapour to be released from the rubber article, on heating and curing which tends to diffuse away from the internal surface of the rubber article (i.e. at the surface of the former), thus reducing the tendency to form blisters or defects from entrapped water vapour, as is sometimes the case with existing technology.

The method of the present invention can also be used in the curing of thermosetting plastics compositions. In many cases, such as epoxy and polyester resins, this involves polymerisation of a monomer or prepolymer to a high molecular weight infusible network structure using catalysts, accelerators and frequently applied heat. Alternatively, as in the thermal vulcanisation of rubbers, the method may involve cross-linking of a previously formed high polymer with unsaturated bonds along the polymer chain, which provide sites for further reaction and chemical bonding between chains.

Thermosetting polymers of the type mentioned above, may contain a wide variety of additives to modify their properties such as glass or carbon fibres to enhance stiffness and strength and/or fillers, which for example, in rubber compositions increase hardness and wear resistance.

The cure times during the processing of thermosetting compositions are accelerated by the method of the present invention, particularly if the polymer or precursor resin is itself microwave-responsive (as is the case for polyester and epoxy resins) or microwave receptive additives (such as carbon black in rubber compositions) are included. Furthermore, by this means, a resin can cure more uniformly, since it is not solely dependent on conducted heat from the surface of the former but also heats up internally. This in turn can result in improved physical properties (e.g. strength) of the composite.

It is desirable that the former should have the following properties:-

It should be sufficiently transparent to microwave energy to allow penetration and heating of the curable composition. It should have sufficient temperature resistance to withstand long-term heating at, and/or repeated heating to, the temperatures required to cure the resin (typically up to 200°C).

It should be chemically resistant to the chemicals in contact with its surface, and possible reaction by-products.

It should be rigid, durable and distortion free under heating temperatures.

It should be wear resistant, particularly in a curing operation in which the thermoset formulation continuously moves across the surface of the former.

Ideally, when placed in a microve field, it should heat up uniformly, without hot spots. (This situation is clearly aided in ceramics having a high thermal conductivity).

## Claims

1. A method of producing an article comprising the steps of:-
   i) covering an external surface of at least a portion of a former (2) with liquid-containing material (5);
   ii) providing heating so as to leave a dry solid coating (6) on said former (2); and
   iii) effecting relative separation between the former (2) and the dry solid coating (6) so as to produce the article
      **characterised in that** the former (2) is a microwave-heatable former which is heated by being subjected to microwave radiation so as to produce the said dry solid coating (6).

2. A method as claimed in claim 1 characterised in that said external surface of the former (2) is made at least predominantly of ceramic material.

3. A method as claimed in claim 2 characterised in that the former (2) is made at least predominantly of microwave receptive glass or silicon carbide.

4. A method as claimed in any preceding claim characterised in that the liquid-containing material (5) is microwave-heatable.

5. A method as claimed in claim 1 or 4 characterised in that the liquid-containing material (5) is a curable or gellable material.

6. A method as claimed in claim 5 characterised in that the liquid-containing material (5) is a latex solution or a plastisol.

7. A method as claimed in claim 5 or 6 characterised in that the microwave radiation is first employed to raise the liquid-containing material (5) to a temperature at which the material (6) becomes substantially solid, the microwave radiation being thereafter employed to raise the substantially solid material (6) to a higher temperature at which it will be cured or gelled.

8. A method as claimed in any preceding claim characterised in that the article is a hollow article.

9. A method as claimed in claim 8 characterised in that the hollow article is made of insulating material, the former (2) being electrically conductive; the hollow article, while still on the former (2), being tested for pinholes therein by maintaining the solid coating (6) on the former (2) in contact with a body of electrically conductive liquid (12) which is at a different potential from that of the former (2), and noting any factor related to a change in the potential difference between the potential of the former (2) and that of the body of liquid (12).

10. A method as claimed in claim 9 characterised in that the testing is carried out by introducing the former (2) with the solid coating (6) thereon, into an earthed tank (11) containing water (12), and applying a voltage to the former (2).

11. A method as claimed in any of claims 8-10 characterised in that the hollow article is a glove or a condom.

12. A method as claimed in any preceding claim characterised in that the former (2) is porous.

13. A method as claimed in claim 12 characterised in that, after the solid coating (6) has been formed on the porous former (2), a gas is passed through the latter so as to assist in removing the solid coating (6) therefrom.

14. Apparatus comprising:-

i) a former (2);

ii) means (3,4) for covering an external surface of at least a portion of the former (2) with liquid-containing material (5);

iii) heating means (7,10) for removing liquid from said material (5) and so as to leave a dry solid coating (6) on said former (2); and

iv) means (16,17) for effecting relative separation between the former (2) and the dry solid coating (6) so as to produce the article;

**characterised in that** the former (2) is a microwave-heatable former and the heating means (7,10) are arranged to heat the former (2) by subjecting it to microwave radiation so as to produce the said dry solid coating (6).

## Patentansprüche

1. Verfahren zur Herstellung eines Gegenstandes, welches die folgenden Schritte aufweist:

i) Abdecken einer äußeren Oberfläche von wenigstens eines Teils eines Formers (2) mit flüssigkeitsenthaltendem Material (5);

ii) Bereitstellen einer Heizung, so daß ein trockener fester Überzug (6) auf dem Former (2) zurückbleibt; und

iii) Bewirken der relativen Trennung zwischen dem Former (2) und dem trockenen festen Überzug (6), so daß der Gegenstand hergestellt wird,

**dadurch gekennzeichnet**, daß der Former (2) ein mikrowellenheizbarer Former ist, welcher erhitzt wird, indem er Mikrowellenstrahlung ausgesetzt wird, so daß der trockene feste Überzug (6) hergestellt wird.

2. Verfahren nach Anspruch 1, dadurch gekennzeichnet, daß die äußere Obefläche des Formers (2) wenigstens überwiegend aus keramischem Material hergestellt wird.

3. Verfahren nach Anspruch 2, dadurch gekennzeichnet, daß der Former (2) wenigstens überwiegend aus mikrowellenempfänglichem Glas oder Siliciumcarbid hergestellt ist.

4. Verfahren nach einem der vorhergehenden Ansprüche, dadurch gekennzeichnet, daß das flüssigkeitsenthaltende Material (5) mikrowellenheizbar ist.

5. Verfahren nach Anspruch 1 oder 2, dadurch gekennzeichnet, daß das flüssigkeitsenthaltende Material (5) ein aushärtbares oder gelierbares Material ist.

6. Verfahren nach Anspruch 5, dadurch gekennzeichnet, daß das flüssigkeitsenthaltende Material (5) eine Latexlösung oder ein Plastisol ist.

7. Verfahren nach Anspruch 5 oder 6, dadurch gekennzeichnet, daß die Mikrowellenstrahlung zuerst angewandt wird, um das flüssigkeitsenthaltene Material (5) auf eine Temperatur zu bringen, bei welcher das Material (6) im wesentlichen fest wird, wobei die Mikrowellenstrahlung danach angewendet wird, um das im wesentlichen feste Material (6) auf eine höhere Temperatur zu bringen, bei welcher es aushärtet oder geliert.

8. Verfahren nach einem der vorgergehenden Ansprüche, dadurch gekennzeichnet, daß der Gegenstand ein hohler Gegenstand ist.

9. Ein Verfahren nach Anspruch 8, dadurch gekennzeichnet, daß der hohle Gegenstand aus isolierendem Material hergestellt ist, wobei der Former (2) elektrisch leitend ist; wobei der hohle Gegenstand, während er noch auf dem Former (2) ist, auf kleine Löcher darin geprüft wird unter Aufrechterhaltung des festen Überzugs (6) auf dem Former (2), in Kontakt mit einem Körper von elektrisch leitender Flüssigkeit (12), die bei einem anderen Potential als das des Formers (2) ist, und wobei irgendein Faktor, der sich auf eine Änderung in der Potentialdifferenz zwischen dem Potential des Formers (2) und dem des Körpers der Flüssigkeit (12) bezieht, festgestellt wird.

10. Verfahren nach Anspruch 9, dadurch gekennzeichnet, daß das Prüfen ausgeführt wird, indem der Former (2) mit dem besten Überzug (6) darauf in einen geerdeten Tank (11), der Wasser (12) enthält, eingeführt wird, und in dem eine Spannung an den Former (2) angelegt wird.

11. Verfahren nach einem der Ansprüche 8-10, dadurch gekennzeichnet, daß der hohle Gegenstand ein Handschuh oder ein Kondom ist.

12. Verfahren nach einem vorhergehenden Ansprüche, dadurch gekennzeichnet, daß der Former (2) porös ist.

13. Verfahren nach Anspruch 12, dadurch gekennzeichnet, daß, nachdem der feste Überzug (6) auf dem porösen Former (2) gebildet worden ist, ein Gas durch den letzteren geleitet wird, so daß die Entfernung des festen Überzugs (6) davon unterstützt wird.

14. Gerät, welches aufweist:

i) einen Former (2);

ii) Einrichtung (3, 4) zum Abdecken einer äußeren Oberfläche von wenigstens einem Teil des Formers (2) mit flüssigkeitsenthaltendem Material (5);

iii) Heizeirichtung (7, 10) zum Entfernen der Flüssigkeit vom Material (5), und so daß ein trockener fester Überzug (6) auf dem Former (2) zurückbleibt; und

iv) Einrichtung (16, 17) zur Beeinsflussung der relativen Trennung zwischen, dem Former (2) und dem trockenen festen Überzug (6), so daß der Gegenstand hergestellt wird;

**dadurch gekennzeichnet**, daß der Former (2) ein mikrowellenheizbarer Former ist, und die Heizeinrichtung (7, 10) angeordnet ist, um den Former (2) zu erhitzen, dadurch daß er Mikrowellenstrahlung augesetzt wird, so daß der trockene feste Überzug (6) hergestellt wird.

## Revendications

1. Procédé de production d'un objet comprenant les stades suivants :

i) recouvrir une surface extérieure d'au moins une portion d'un gabarit (2) avec un matériau contenant un liquide (5) ;

ii) chauffer de façon à laisser un revêtement solide sec (6) sur le gabarit (2) ; et

iii) séparer l'un de l'autre le gabarit (2) et le revêtement solide sec (6) de façon à produire l'objet,

**caractérisé en ce que** le gabarit (2) est un gabarit pouvant être chauffé par hyperfréquences, lequel est chauffé en étant soumis à un rayonnement hyperfréquences de façon à produire le revêtement solide sec (6).

2. Procédé selon la revendication 1, caractérisé en ce que la surface extérieure du gabarit (2) est, tout au moins de façon principale, en un matériau céramique.

3. Procédé selon la revendication 2, caractérisé en ce que le gabarit (2) est, au moins de façon principale, en un verre sensible aux hyperfréquences ou en carbure de silicium.

4. Procédé selon l'une des revendications précédentes, caractérisé en ce que le matériau contenant un liquide (5) peut être chauffé par hyperfréquences.

5. Procédé selon la revendication 1 ou la revendication 4, caractérisé en ce que le matériau contenant un liquide (5) est un matériau durcissable ou gélifiable.

6. Procédé selon la revendication 5, caractérisé en ce que le matériau contenant un liquide (5) est une solution de latex ou un plastisol.

7. Procédé selon la revendication 5 ou la revendication 6, caractérisé en ce que le rayonnement hyperfréquences est tout d'abord utilisé pour élever le matériau contenant un liquide (5) à une température à laquelle le matériau devient pratiquement solide, le rayonnement hyperfréquences étant ensuite utilisé pour élever le matériau pratiquement solide (6) à une température supérieure à laquelle il prendra ou se gélifiera.

8. Procédé selon l'une des revendications précédentes, caractérisé en ce que l'objet est un objet creux.

9. Procédé selon la revendication 8, caractérisé en ce que l'objet creux est en un matériau isolant, le gabarit (2) étant électriquement conducteur et en ce qu'on contrôle les piqûres de cet objet creux, pendant qu'il est encore sur le gabarit (2), en maintenant le revêtement solide (6) sur la gabarit (2) en contact avec une masse d'un liquide électriquement conducteur (12) qui est à un potentiel différent de celui du gabarit (2) et en notant tout facteur relatif à un changement dans la différence de potentiel entre le potentiel du gabarit (2) et celui de la masse de liquide (12).

10. Procédé selon la revendication 9, caractérisé en ce que le contrôle est effectué en introduisant le gabarit (2) portant le revêtement solide (6) dans une cuve mise à la terre (11) contenant de l'eau (12) et en appliquant une tension au gabarit (2).

11. Procédé selon l'une des revendications 8 à 10, caractérisé en ce que l'objet creux est un gant ou un condom.

12. Procédé selon l'une des revendications précédentes, caractérisé en ce que le gabarit (2) est poreux.

13. Procédé selon la revendication 12, caractérisé en ce que, après que le revêtement solide (6) a été formé sur le gabarit poreux (2), on fait passer un gaz à travers ce dernier pour aider à en retirer le revêtement solide (6).

14. Dispositif comprenant :

i) un gabarit (2) ;

ii) des moyens (3,4) pour recouvrir une surface extérieure d'au moins une portion du gabarit (2) avec un matériau contenant un liquide (5) ;

iii) des moyens de chauffage (7,10) pour éliminer le liquide du matériau (5) et laisser un revêtement solide sec (6) sur le gabarit (2) ; et

iv) des moyens (16,17) pour séparer l'un de l'autre le gabarit (2) et le revêtement solide sec (6) de façon à produire l'objet,

**caractérisé en ce que** le gabarit (2) est un gabarit pouvant être chauffé aux hyperfréquences et que les moyens de chauffage (7,10) sont agencés pour chauffer le gabarit (2) en le soumettant à un rayonnement hyperfréquences de façon à produire ledit revêtement solide sec (6).

A          B          C          D